# FASCICULE DE BREVET EUROPEEN

(11) **EP 3 750 873 B1**
(45) Date de publication et mention de la délivrance du brevet: **20.12.2023**
(21) Numéro de dépôt: 20179342.9
(22) Date de dépôt: 10.06.2020
(51) Int. Cl.: C07C 303/32, C07C 309/15

(54) **PROCÉDÉ DE FABRICATION DE N-ACÉTYL-TAURINATE DE MAGNÉSIUM**
VERFAHREN ZUR HERSTELLUNG VON MAGNESIUM-N-ACETYL-TAURAT
METHOD FOR MANUFACTURING MAGNESIUM N-ACETYL-TAURINATE

(30) Priorité: 11.06.2019 BE 201905372
(43) Date de publication de la demande: 16.12.2020
(73) Titulaire: Synapharm Industrial Synthesis, 4432 Alleur (BE)
(72) Inventeur: Danhier, Philippe, 7382 Audregnies (BE); Azzam, Pascale, 4432 Alleur (BE)
(74) Mandataire: Gevers Patents

(56) Documents cités:
- FR-A1- 2 384 751
- ARFUZIR N N N ET AL: "Protective effect of magnesium acetyltaurate against endothelin-induced retinal and optic nerve injury", NEUROSCIENCE, vol. 325, 2016, pages 153-164, XP029522821, ISSN: 0306-4522, DOI: 10.1016/J.NEUROSCIENCE.2016.03.041

## Description

La présente invention porte sur un procédé de fabrication de N-acétyl-taurinate de magnésium, en particulier sur un procédé de fabrication de N-acétyl-taurinate de magnésium dihydraté.

Les sels de N-acétyl-taurine sont essentiellement obtenus selon un procédé similaire à celui de la préparation d'acétyl-taurinate de sodium tel que décrit par Teracoka (Teracoka, Hoope-Seyler Zeitschrift für Physiologische Chemie, 145, 1925, 242), c'est-à-dire par action d'anhydre acétique sur la taurine en présence d'une base correspondant au sel à obtenir au point d'ébullition du mélange. Le N-acétyl-taurinate de magnésium est obtenu par acétylation de la taurine (un acide aminé soufré) en présence d'un composé magnésien, par exemple en présence d'hydroxyde ou d'oxyde de magnésium. L'acétylation s'effectue sur l'azote de la taurine (fonction NH₂), ce qui enlève la caractère zwitterion, seule la charge négative de la fonction acide sulfonique (SOsH) subsistant. Cette particularité rend le N-acétyl-taurinate de magnésium plus lipophile et facilite notamment son passage dans les phospholipides membranaires neuronaux. Les sels de N-acétyl-taurine permettent d'exploiter de nouvelles propriétés de la taurine et augmentent en particulier son pouvoir de pénétration cellulaire, ceci en augmentant les activités neuro-musculaires de l'acide 2-aminoéthanesulfonique. En particulier, l'acétylation permet d'augmenter la pénétration cellulaire de nombreux composés organiques sulfurés sans en altérer les activités biologiques.

Le document FR2384751 décrit un procédé de fabrication selon lequel le N-acétyl-taurinate de magnésium est obtenu par mélange de magnésie, de taurine, d'eau et d'acide acétique avant deux étapes successives de séchage, l'une sous vide à 100°C et l'autre avec un solvant de séchage, de telle sorte à obtenir une cristallisation lente par refroidissement à température ambiante. Le rendement de ce procédé de fabrication par rapport au magnésium engagé est de l'ordre de 65% ([quantité en poids de magnésie mise en oeuvre / quantité en poids de N-acétyl-taurinate de magnésium obtenue à l'issue du procédé de fabrication] * 100).

Outre le procédé de fabrication de N-acétyl-taurinate de magnésium décrit dans le document FR2384751, le document de Arfuzir et al. (Arfuzir et al., Protective effect of magnésium acetyltaurate against endothelin-induced retinal and optic nerve injury, Neuroscience 325, 2016, 153-164) décrit un autre procédé de fabrication de N-acétyl-taurinate de magnésium. Les étapes de ce procédé sont les suivantes : **(a)** mélange de taurine et d'oxyde de magnésium dans de l'eau ; **(b)** agitation du mélange obtenu à l'étape (a) à une température de 80-90°C durant 10 min avec ajout d'anhydre acétique; **(c)** agitation du mélange obtenu à l'étape (b) à une température de 80-90°C durant 30 min ; **(d)** évaporation sous vide du mélange obtenu à l'étape (c) ; **(e)** mise en contact du résidu obtenu par évaporation à l'étape (d) avec de l'éthanol (alcool éthylique) et refroidissement au réfrigérateur durant 24h pour obtenir un précipité ; **(f)** filtration du précipité obtenu à l'étape (e) et lavage à froid de ce dernier successivement avec de l'éthanol (alcool éthylique), de l'acétone et de l'éther diéthylique ; **(g)** séchage à l'air à température ambiante du précipité lavé obtenu à l'étape (f) puis à 40-50°C sous vide pour finalement obtenir du N-acétyl-taurinate de magnésium anhydre sous forme d'une poudre présentant un poids moléculaire de 356,656 (formule C₈H₁₆MgN₂O₈S₂). Le rendement de ce procédé par rapport au magnésium engagé est de l'ordre de 90% ([quantité en poids d'oxyde de magnésium mise en oeuvre / quantité en poids de N-acétyl-taurinate de magnésium obtenue à l'issue du procédé de fabrication] * 100).

Le N-acétyl-taurinate de magnésium (C₈H₁₆MgN₂O₈S₂) est notamment connu pour ses propriétés cyto-vasculo protectrices telles que des propriétés anti-aggrégantes plaquettaires et protectrices des thromboses veineuses et artérielles ainsi qu'une action stabilisatrice de la membrane érythrocytaire. En outre, un effet protectif du magnésium N-acétyl-taurinate contre le glaucome en agissant sur la dérégulation vasculaire a été démontré, tout comme les propriétés angio-protectrices du N-acétyl-taurinate de magnésium par son action anti-inflammatoire en restaurant les teneurs en eNOS (endothélial Nitric Oxide Synthase 3). Des études ont également démontré un effet bénéfique du magnésium N-acétyl-taurinate sur la neurotoxicité due à l'hyperexcitabilité des récepteurs glutamatergiques ionotropes. En effet, le magnésium N-acétyl-taurinate a une action intraneuronale non seulement sur le récepteur NMDA mais également sur les deux autres récepteurs ionotropes de l'acide glutamique à savoir les récepteurs AMPA et KA, ceux-ci étant impliqués dans la vitesse de la transmission synaptique. Il a été remarqué qu'un tel composé présente une analogie structurelle avec l'acide glutamique mais également l'acide kaïnique. Ainsi, le N-acétyl-taurinate de magnésium va cibler l'ensemble des récepteurs glutamatergiques NMDAR, AMPAR et KAR entrainant par conséquent l'inhibition des voies de signalisation en aval de ces récepteurs.

Malheureusement, même si le N-acétyl-taurinate de magnésium présente de nombreuses propriétés intéressantes, le procédé de fabrication de N-acétyl-taurinate de magnésium décrit dans le document FR2384751 ne donne lieu qu'à un rendement de l'ordre de 65% par rapport au magnésium engagé tandis que le procédé de Arfuzir et al. met en oeuvre de l'éthanol (alcool éthylique), de l'acétone et de l'éther diéthylique lors de lavages successifs, ces intrants organiques donnant lieu à des rejets toxiques dommageables pour l'environnement et étant dangereux à manipuler par l'opérateur réalisant la synthèse de N-acétyl-taurinate de magnésium. Par ailleurs, même si le procédé de fabrication de N-acétyl-taurinate de magnésium décrit dans le document de Arfuzir et al. donne lieu à un rendement de l'ordre de 90% par rapport au magnésium engagé, il requière toutefois une longue étape de refroidissement d'une durée d'au moins 24h mais aussi des étapes de chauffage à des températures élevées (>50°C). Le procédé selon Arfuzir et al. est donc consommateur d'énergie pour pouvoir assurer un refroidissement important et des chauffages à des températures élevées, ce qui affecte le rendement global de ce procédé.

Il existe donc à ce jour un besoin de procurer un procédé rentable de fabrication de N-acétyl-taurinate de magnésium qui réduit les intrants organiques mis en oeuvre, qui soit plus respectueux de l'environnement (réduction des rejets toxiques et réduction de l'énergie requise), qui permette d'obtenir du N-acétyl-taurinate de magnésium de façon plus rapide, qui réduit les expositions de l'opérateur à des substances toxiques et dangereuses et dont le rendement est au moins équivalent voire supérieur aux rendements obtenus avec les procédés actuels.

Pour résoudre au moins en partie ces problèmes, il est prévu suivant l'invention, un procédé de fabrication de N-acétyl-taurinate de magnésium, en particulier un procédé de fabrication de N-acétyl-taurinate de magnésium dihydraté, comprenant :
**a)** une étape de solubilisation (i) de taurine, (ii) d'au moins un composé magnésien, par exemple d'oxyde de magnésium ou d'hydroxyde de magnésium et (iii) d'anhydre acétique dans de l'eau avec salification et acétylation de ladite taurine pour obtenir une solution comprenant de la taurine magnésique sous forme acétylée ;
**b)** une étape d'évaporation et/ou de distillation de ladite solution obtenue à l'étape **a)** pour éliminer au moins partiellement les excès d'eau et d'acide acétique et obtenir un premier précipité, en particulier un premier précipité sous forme d'un distillat, comprenant la taurine magnésique sous forme acétylée ;
**c)** au moins une étape de lavage à l'eau, par exemple par filtration, dudit premier précipité obtenu à l'étape **b),** ladite étape de lavage à l'eau étant suivie par une étape **d)** d'évaporation et/ou de distillation pour éliminer au moins partiellement les excès d'eau et d'acide acétique et obtenir un deuxième précipité, en particulier un deuxième précipité sous forme d'un distillat, comprenant la taurine magnésique sous forme acétylée;
**e)** au moins une étape de lavage, avec une solution de lavage comprenant de l'éthanol, de préférence avec une solution de lavage comprenant uniquement de l'éthanol, dudit deuxième précipité obtenu à l'étape **d),** ladite étape de lavage avec une solution de lavage comprenant de l'éthanol, de préférence avec une solution de lavage comprenant uniquement de l'éthanol, étant suivie par une étape **f)** d'évaporation et/ou de distillation pour éliminer au moins partiellement les excès d'eau et d'éthanol et obtenir un troisième précipité comprenant la taurine magnésique sous forme acétylée ;
**g)** une étape de refroidissement dudit troisième précipité obtenu à l'étape **f)** ;
**h)** une étape de purification et/ou de lavage dudit troisième précipité refroidi obtenu à l'étape **g)** pour obtenir un quatrième précipité sous forme d'un gâteau ; et
**i)** une étape de séchage/dessiccation dudit quatrième précipité sous forme d'un gâteau purifié et/ou lavé obtenu à l'étape **h).**

Le procédé selon la présente invention ne met en oeuvre que de l'éthanol ou de l'eau lors des étapes de lavage, ce qui permet de réduire considérablement les intrants organiques et par conséquent les rejets toxiques et dommageables pour l'environnement, ceci d'autant plus que l'éthanol rejeté peut être réutilisé pour d'autres étapes de lavages lorsque le procédé est répété. En outre, le procédé suivant la présente invention présente un rendement de l'ordre de 92% par rapport au magnésium engagé et ne requière pas une étape de refroidissement d'une durée d'au moins 24h : le procédé selon l'invention présente donc un rendement amélioré par rapport aux procédés connus de l'état de la technique et permet de réduire significativement l'énergie requise pour obtenir de N-acétyl-taurinate de magnésium.

De façon avantageuse, selon l'invention, le N-acétyl-taurinate de magnésium obtenu est du N-acétyl-taurinate de magnésium dihydraté comprenant deux molécules d'eau intrinsèques. Par les termes « deux molécules d'eau intrinsèques », il est entendu, au sens de la présente invention, que les deux molécules d'eau sont inhérentes au N-acétyl-taurinate de magnésium dihydraté, c'est-à-dire qu'elles font partie intégrante du N-acétyl-taurinate de magnésium dihydraté, à la différence de l'eau d'hydratation qui pourrait être absorbée ou adsorbée par ce composé. Le N-acétyl-taurinate de magnésium dihydraté (C₈H₂₀MgN₂O₁₀S₂), dénommé également magnésium N-acétyl-taurinate dihydraté, est un vecteur magnésique et un analogue magnésique de la taurine qui présente un poids moléculaire de 392,677 g/mol, deux molécules d'eau (H₂O) étant intrinsèques à la molécule de N-acétyl-taurinate de magnésium dihydraté. Le poids moléculaire du N-acétyl-taurinate de magnésium dihydraté diffère de celui du N-acétyl-taurinate de magnésium non dihydraté (C₈H₁₆MgN₂O₈S₂) qui est lui de 356,656 g/mol.

Comme le N-acétyl-taurinate de magnésium non dihydraté, le N-acétyl-taurinate de magnésium dihydraté comprenant deux molécules d'eau intrinsèques possède différentes caractéristiques telles qu'un dérivé β aminé soufré, un acide sulfonique (non carboxylique), un N-acétylé, ne présente pas le caractère amphotère de la taurine (forme Zwitterion, c'est-à-dire qu'une charge positive et une charge négative se trouvent sur le même résidu) et optimise le caractère taurinergique intracellulaire. La charge électrique sur l'azote de la taurine étant supprimée par l'acétylation, seuls les électrons du cation Mg⁺⁺ sont maintenus chélatés par les deux radicaux sulfoniques de la taurine. Cela conduit à un dérivé éthanamide (acétamide) au caractère lipophile plus marqué que celui de la taurine amphotère, ce qui facilite la pénétration au travers les phospholipides membranaires neuronaux. Les dérivés éthanamides (acétamides) caractérisent les molécules utilisées pour leurs actions nootropes (piracetam-like), anti-convulsivantes et anti-épileptiques (levitracetam-like).

Selon un mode de réalisation suivant l'invention, ladite étape **a)** de solubilisation dans de l'eau est effectuée par mise en solution simultanée ou différée dans le temps de ladite taurine, dudit composé magnésien (par exemple d'oxyde de magnésium ou d'hydroxyde de magnésium) et dudit anhydre acétique. Par exemple, la taurine et le composé magnésien peuvent être solubilisés dans l'eau dans un premier temps pour obtenir une première solution avant d'y ajouter, dans un deuxième temps, l'anhydre acétique (par exemple de façon progressive par coulée exothermique) pour obtenir une deuxième solution comprenant alors de la taurine magnésique sous forme acétylée.

De préférence, selon le procédé de fabrication de N-acétyl-taurinate de magnésium suivant l'invention, l'étape **h)** de purification et/ou de lavage dudit troisième précipité est effectuée par filtration en présence d'une solution de filtration, de préférence en présence d'une solution de filtration comprenant de l'éthanol.

Avantageusement, selon le procédé de fabrication de N-acétyl-taurinate de magnésium suivant l'invention, l'étape **h)** de purification et/ou de lavage dudit troisième précipité est effectuée par essorage centrifuge en présence d'une solution d'essorage, de préférence en présence d'une solution d'essorage comprenant de l'éthanol.

Préférentiellement, selon le procédé de fabrication de N-acétyl-taurinate de magnésium suivant l'invention, l'étape **h)** de purification et/ou de lavage dudit troisième précipité par essorage centrifuge est réalisée à une vitesse d'essorage centrifuge comprise entre 500 et 1500 tours/minute, de préférence à une vitesse d'essorage centrifuge comprise entre 800 et 1200 tours/minute.

Avantageusement, selon le procédé de fabrication de N-acétyl-taurinate de magnésium suivant l'invention, l'étape **a)** de solubilisation est réalisée en présence d'une solution de solubilisation comprenant de l'éthanol.

Préférentiellement, selon le procédé de fabrication de N-acétyl-taurinate de magnésium suivant l'invention, l'étape **a)** et/ou l'étape **b)** et/ou l'étape **d)** et/ou l'étape **f)** et/ou l'étape **h)** et/ou l'étape **i)** est réalisée sous vide. En particulier, dans le cadre de la présente invention, il a été déterminé que lorsque l'étape **a)** et/ou l'étape **b)** et/ou l'étape **d)** et/ou l'étape **f)** et/ou l'étape **h)** et/ou l'étape **i)** est réalisée sous vide, le rendement du procédé est augmenté par rapport au magnésium engagé. Selon l'invention, de l'azote ou tout autre gaz inerte peut être utilisé afin de créer le vide. Également, toute technique permettant d'obtenir le vide peut être employée dans le cadre de la présente invention. En particulier, lorsqu'au moins l'une des étapes **a), b), d), f), h)** ou **i)** est réalisée sous vide, la cinétique de réaction d'acétylation du procédé selon l'invention est nettement favorisée par déplacement, selon le principe de Le Chatelier, de l'équilibre chimique en faveur de l'acétylation.

De préférence, selon le procédé de fabrication de N-acétyl-taurinate de magnésium suivant l'invention, l'étape **a)** et/ou l'étape **b)** et/ou l'étape **c)** et/ou l'étape **d)** et/ou l'étape **e)** et/ou l'étape **f)** et/ou l'étape **g)** et/ou l'étape **h)** et/ou l'étape **i)** est réalisée à une température inférieure ou égale à 50°C. En particulier, dans le cadre de la présente invention, il a été déterminé que lorsque l'étape **a)** et/ou l'étape **b)** et/ou l'étape **c)** et/ou l'étape **d)** et/ou l'étape **e)** et/ou l'étape **f)** et/ou l'étape **g)** et/ou l'étape **h)** et/ou l'étape **i)** est réalisée à une température inférieure ou égale à 50°C, le rendement du procédé est augmenté par rapport au magnésium engagé. En particulier, lorsqu'au moins l'une des étapes **a), b), c), d), e), f), g), h)** ou **i)** est réalisée à une température inférieure ou égale à 50°C, la cinétique de réaction d'acétylation du procédé selon l'invention est nettement favorisée par déplacement, selon le principe de Le Chatelier, de l'équilibre chimique en faveur de l'acétylation.

Selon un mode de réalisation préféré de l'invention, l'étape **a)** de solubilisation est réalisée sous vide et à une température inférieure ou égale à 50°C. Il a été montré que de la sorte la cinétique de réaction d'acétylation du procédé selon l'invention est nettement favorisée par déplacement, selon le principe de Le Chatelier, de l'équilibre chimique en faveur de l'acétylation et que la vitesse de réaction est optimisée. La solubilisation sous vide à une température inférieure ou égale à 50°C permet plus particulièrement de faciliter l'extraction de l'acide acétique gazeux produit en excès par hydrolyse de l'anhydre acétique dans l'eau, ceci en faveur de la réaction d'acétylation.

Selon un autre mode de réalisation préféré suivant l'invention, les étapes **a)** de solubilisation, **h)** de purification et/ou de lavage dudit troisième précipité refroidi et **i)** de séchage/dessiccation dudit quatrième précipité sous forme d'un gâteau purifié et/ou lavé sont réalisées sous vide et à une température inférieure ou égale à 50°C. Outre le fait que la cinétique de réaction d'acétylation du procédé selon l'invention est nettement favorisée par déplacement, selon le principe de Le Chatelier, de l'équilibre chimique en faveur de l'acétylation et que la vitesse de réaction est optimisée dans de telles conditions, il a aussi été montré que de la sorte le rendement global du procédé selon l'invention est d'autant plus optimisé.

Selon encore un autre mode de réalisation préféré suivant l'invention, les étapes **a)** de solubilisation, **h)** de purification et/ou de lavage dudit troisième précipité refroidi par essorage centrifuge en présence d'une solution d'essorage, de préférence en présence d'une solution d'essorage comprenant de l'éthanol et **i)** de séchage/dessiccation dudit quatrième précipité sous forme d'un gâteau purifié et/ou lavé sont réalisées sous vide et à une température inférieure ou égale à 50°C. Outre le fait que la cinétique de réaction d'acétylation du procédé selon l'invention est nettement favorisée par déplacement, selon le principe de Le Chatelier, de l'équilibre chimique en faveur de l'acétylation dans de telles conditions, il a aussi été montré que de la sorte le rendement global du procédé selon l'invention est encore plus optimisé.

Selon l'invention, ladite étape de séchage/dessiccation dudit quatrième précipité sous forme d'un gâteau purifié et/ou lavé peut être réalisée par filtration.

D'autres caractéristiques, détails et avantages de l'invention ressortiront des exemples donnés ci-après, à titre non limitatif.

### Exemple comparatif

Le procédé suivant la présente invention a été comparé aux procédés respectivement décrits dans le document FR2384751 et dans le document de Arfuzir et al., ceci en termes de rendement par rapport au magnésium engagé. Plus particulièrement, le procédé suivant selon l'invention a été comparé à ces deux procédés connus de l'état de la technique :
**a)** solubilisation, sous vide et à une température de 50°C, (i) de 0,2 M de taurine, (ii) de 0,102 M d'hydroxyde de magnésium et (iii) de 0,265 M d'anhydre acétique dans de l'eau avec salification et acétylation de la taurine pour obtenir une solution comprenant de la taurine magnésique sous forme acétylée ;
**b)** distillation sous vide et à une température de 50°C de la solution obtenue à l'étape **a)** pour éliminer au moins partiellement les excès d'eau et d'acide acétique et obtenir un premier précipité sous forme d'un distillat comprenant la taurine magnésique sous forme acétylée ;
**c)** lavage à l'eau par filtration, du premier précipité obtenu à l'étape **b),** l'étape de lavage à l'eau étant suivie par une étape **d)** de distillation sous vide et à une température de 50°C pour éliminer au moins partiellement les excès d'eau et d'acide acétique et obtenir un deuxième précipité sous forme d'un distillat comprenant la taurine magnésique sous forme acétylée;
**e)** lavage, avec une solution de lavage comprenant uniquement de l'éthanol, du deuxième précipité obtenu à l'étape **d),** l'étape de lavage avec une solution de lavage comprenant uniquement de l'éthanol étant suivie par une étape **f)** de distillation sous vide et à une température de 50°C pour éliminer au moins partiellement les excès d'eau et d'éthanol et obtenir un troisième précipité comprenant la taurine magnésique sous forme acétylée ;
**g)** refroidissement à une température d'environ 20 à 25°C du troisième précipité obtenu à l'étape **f)** ;
**h)** purification, par essorage centrifuge sous vide et à une température de 50°C à une vitesse d'essorage centrifuge de 800 à 1200 tours/minute, du troisième précipité refroidi obtenu à l'étape **g)** pour obtenir un quatrième précipité sous forme d'un gâteau ; et
**i)** une étape de séchage/dessiccation à une température de 50°C du quatrième précipité sous forme d'un gâteau purifié et/ou lavé obtenu à l'étape **h).**

Pour chacun des procédés, les rendements obtenus par rapport au magnésium engagé sont présentés au tableau 1 ci-après. Ces rendements ont été calculé comme suit : [quantité en poids du composé magnésien mise en oeuvre / quantité en poids de N-acétyl-taurinate de magnésium obtenue à l'issue du procédé de fabrication] * 100.

**Tableau 1**

| | Rendement (%) |
|---|---|
| Procédé selon FR2384751 | 65 |
| Procédé selon Arfuzir et al. | 90 |
| Procédé selon l'invention | 92 |

Comme on peut aisément le constater, le procédé suivant l'invention permet d'optimiser le rendement par rapport au magnésium engagé par rapport aux deux procédés connus de l'état de la technique. Par ailleurs, au contraire du procédé d'Arfuzir et al., le procédé suivant l'invention ne met en oeuvre que de l'eau et un seul intrant organique (l'éthanol) pour les étapes de lavage au lieu de trois intrants organiques (éthanol + acétone + éther diéthylique).

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Procédé de fabrication de N-acétyl-taurinate de magnésium, en particulier de N-acétyl-taurinate de magnésium dihydraté, comprenant :
**a)** une étape de solubilisation (i) de taurine, (ii) d'au moins un composé magnésien, par exemple d'oxyde de magnésium ou d'hydroxyde de magnésium et (iii) d'anhydre acétique dans de l'eau avec salification et acétylation de ladite taurine pour obtenir une solution comprenant de la taurine magnésique sous forme acétylée ;
**b)** une étape d'évaporation et/ou de distillation de ladite solution obtenue à l'étape **a)** pour éliminer au moins partiellement les excès d'eau et d'acide acétique et obtenir un premier précipité, en particulier un premier précipité sous forme d'un distillat, comprenant la taurine magnésique sous forme acétylée ;
**c)** au moins une étape de lavage à l'eau, par exemple par filtration, dudit premier précipité obtenu à l'étape **b),** ladite étape de lavage à l'eau étant suivie par une étape **d)** d'évaporation et/ou de distillation pour éliminer au moins partiellement les excès d'eau et d'acide acétique et obtenir un deuxième précipité, en particulier un deuxième précipité sous forme d'un distillat, comprenant la taurine magnésique sous forme acétylée;
**e)** au moins une étape de lavage, avec une solution de lavage comprenant de l'éthanol, de préférence avec une solution de lavage comprenant uniquement de l'éthanol, dudit deuxième précipité obtenu à l'étape **d),** ladite étape de lavage avec une solution de lavage comprenant de l'éthanol, de préférence avec une solution de lavage comprenant uniquement de l'éthanol, étant suivie par une étape **f)** d'évaporation et/ou de distillation pour éliminer au moins partiellement les excès d'eau et d'éthanol et obtenir un troisième précipité comprenant la taurine magnésique sous forme acétylée ;
**g)** une étape de refroidissement dudit troisième précipité obtenu à l'étape **f)** ;
**h)** une étape de purification et/ou de lavage dudit troisième précipité refroidi obtenu à l'étape **g)** pour obtenir un quatrième précipité sous forme d'un gâteau ; et
**i)** une étape de séchage/dessiccation dudit quatrième précipité sous forme d'un gâteau purifié et/ou lavé obtenu à l'étape **h).**

2. Procédé de fabrication de N-acétyl-taurinate de magnésium selon la revendication 1, dans lequel ladite étape **a)** de solubilisation dans de l'eau est effectuée par mise en solution simultanée ou différée dans le temps de ladite taurine, dudit oxyde de magnésium ou dudit hydroxyde de magnésium et dudit anhydre acétique.

3. Procédé de fabrication de N-acétyl-taurinate de magnésium selon la revendication 1 ou 2, dans lequel ladite étape **h)** de purification et/ou de lavage dudit troisième précipité est effectuée par filtration en présence d'une solution de filtration, de préférence en présence d'une solution de filtration comprenant de l'éthanol.

4. Procédé de fabrication de N-acétyl-taurinate de magnésium selon la revendication 1 ou 2, dans lequel ladite étape **h)** de purification et/ou de lavage dudit troisième précipité est effectuée par essorage centrifuge en présence d'une solution d'essorage, de préférence en présence d'une solution d'essorage comprenant de l'éthanol.

5. Procédé de fabrication de N-acétyl-taurinate de magnésium selon la revendication 4, dans lequel ladite étape **h)** de purification et/ou de lavage dudit troisième précipité par essorage centrifuge est réalisée à une vitesse d'essorage centrifuge comprise entre 500 et 1500 tours/minute, de préférence à une vitesse d'essorage centrifuge comprise entre 800 et 1200 tours/minute.

6. Procédé de fabrication de N-acétyl-taurinate de magnésium selon l'une quelconque des revendications précédentes, dans lequel ladite étape **a)** de solubilisation est réalisée en présence d'une solution de solubilisation comprenant de l'éthanol.

7. Procédé de fabrication de N-acétyl-taurinate de magnésium selon l'une quelconque des revendications précédentes, dans lequel ladite étape **a)** et/ou ladite étape **b)** et/ou ladite étape **d)** et/ou ladite étape **f)** et/ou ladite étape **h)** et/ou ladite étape **i)** est réalisée sous vide.

8. Procédé de fabrication de N-acétyl-taurinate de magnésium selon l'une quelconque des revendications précédentes, dans lequel ladite étape **a)** et/ou ladite étape **b)** et/ou ladite étape **c)** et/ou ladite étape **d)** et/ou ladite étape **e)** et/ou ladite étape **f)** et/ou ladite étape **g)** et/ou ladite étape **h)** et/ou ladite étape **i)** est réalisée à une température inférieure ou égale à 50°C.

9. Procédé de fabrication de N-acétyl-taurinate de magnésium selon l'une quelconque des revendications précédentes, dans lequel ladite étape **a)** de solubilisation est réalisée sous vide et à une température inférieure ou égale à 50°C.

10. Procédé de fabrication de N-acétyl-taurinate de magnésium selon l'une quelconque des revendications précédentes, dans lequel les étapes **a)** de solubilisation, **h)** de purification et/ou de lavage dudit troisième précipité refroidi et **i)** de séchage/dessiccation dudit quatrième précipité sous forme d'un gâteau purifié et/ou lavé sont réalisées sous vide et à une température inférieure ou égale à 50°C.

11. Procédé de fabrication de N-acétyl-taurinate de magnésium selon l'une quelconque des revendications précédentes, dans lequel les étapes **a)** de solubilisation, **h)** de purification et/ou de lavage dudit troisième précipité refroidi par essorage centrifuge en présence d'une solution d'essorage, de préférence en présence d'une solution d'essorage comprenant de l'éthanol et **i)** de séchage/dessiccation dudit quatrième précipité sous forme d'un gâteau purifié et/ou lavé sont réalisées sous vide et à une température inférieure ou égale à 50°C.

## Patentansprüche

1. Verfahren zur Herstellung von Magnesium-N-acetyltaurinat, insbesondere Magnesium-N-acetyltaurinatdihydrat, umfassend:
**a)** einen Solubilisierungsschritt (i) von Taurin, (ii) von mindestens einer Magnesiumverbindung, zum Beispiel Magnesiumoxid oder Magnesiumhydroxid und (iii) von Essigsäureanhydrid in Wasser unter Salzbildung und Acetylierung des Taurins, um eine Lösung zu erhalten, die magnesisches Taurin in acetylierter Form umfasst;
**b)** einen Verdampfungs- und/oder Destillationsschritt der in Schritt **a)** erhaltenen Lösung, um die Überschüsse an Wasser und Essigsäure mindestens teilweise zu entfernen und ein erstes Präzipitat zu erhalten, insbesondere ein erstes Präzipitat in Form eines Destillats, umfassend das magnesische Taurin in acetylierter Form;
**c)** mindestens einen Waschschritt mit Wasser, zum Beispiel durch Filtration, des in dem Schritt **b)** erhaltenen ersten Präzipitats, wobei der Waschschritt mit Wasser gefolgt wird von einem Schritt **d)** der Verdampfung und/oder Destillation, um die Überschüsse an Wasser und Essigsäure mindestens teilweise zu entfernen und ein zweites Präzipitat zu erhalten, insbesondere ein zweites Präzipitat in Form eines Destillats, umfassend das magnesische Taurin in acetylierter Form;
**e)** mindestens einen Waschschritt mit einer Waschlösung umfassend Ethanol, vorzugsweise mit einer Waschlösung, die einzig Ethanol umfasst, des in Schritt **d)** erhaltenen zweiten Präzipitats, wobei der Waschschritt mit einer Waschlösung umfassend Ethanol, vorzugsweise mit einer Waschlösung, die einzig Ethanol umfasst, gefolgt wird von einem Schritt **f)** der Verdampfung und/oder Destillation, um die Überschüsse an Wasser und Ethanol mindestens teilweise zu entfernen und ein drittes Präzipitat zu erhalten, umfassend das magnesische Taurin in acetylierter Form;
**g)** einen Schritt der Kühlung des in Schritt **f)** erhaltenen dritten Präzipitats;
**h)** einen Schritt der Reinigung und/oder des Waschens des in Schritt **g)** erhaltenen gekühlten dritten Präzipitats, um ein viertes Präzipitat in Form eines Kuchens zu erhalten; und
**i)** einen Schritt der Trocknung/Austrocknung des vierten Präzipitats in Form eines in Schritt **h)** erhaltenen gereinigten und/oder gewaschenen Kuchens.

2. Verfahren zur Herstellung von Magnesium-N-acetyltaurinat nach Anspruch 1, wobei der Schritt a) der Solubilisierung in Wasser durch gleichzeitiges oder zeitlich verzögertes In-Lösung-Bringen des Taurins, des Magnesiumoxids oder des Magnesiumhydroxids und des Essigsäureanhydrids erfolgt.

3. Verfahren zur Herstellung von Magnesium-N-acetyltaurinat nach Anspruch 1 oder 2, wobei der Schritt h) der Reinigung und/oder des Waschens des dritten Präzipitats durch Filtration in Gegenwart einer Filtrationslösung, vorzugsweise in Gegenwart einer Filtrationslösung umfassend Ethanol, erfolgt.

4. Verfahren zur Herstellung von Magnesium-N-acetyltaurinat nach Anspruch 1 oder 2, wobei der Schritt **h)** der Reinigung und/oder des Waschens des dritten Präzipitats durch Zentrifugieren in Gegenwart einer Entwässerungslösung, vorzugsweise in Gegenwart einer Entwässerungslösung umfassend Ethanol, erfolgt.

5. Verfahren zur Herstellung von Magnesium-N-acetyltaurinat nach Anspruch 4, wobei der Schritt **h)** der Reinigung und/oder des Waschens des dritten Präzipitats durch Zentrifugieren bei einer Zentrifugiergeschwindigkeit zwischen 500 und 1500 Umdrehungen/Minute, vorzugsweise bei einer Zentrifugiergeschwindigkeit zwischen 800 und 1200 Umdrehungen/Minute durchgeführt wird.

6. Verfahren zur Herstellung von Magnesium-N-acetyltaurinat nach irgendeinem der vorstehenden Ansprüche, wobei der Schritt **a)** der Solubilisierung in Gegenwart einer Solubilisierungslösung umfassend Ethanol durchgeführt wird.

7. Verfahren zur Herstellung von Magnesium-N-acetyltaurinat nach irgendeinem der vorstehenden Ansprüche, wobei der Schritt **a)** und/oder der Schritt **b)** und/oder der Schritt **d)** und/oder der Schritt **f)** und/oder der Schritt **h)** und/oder der Schritt **i)** unter Vakuum durchgeführt wird.

8. Verfahren zur Herstellung von Magnesium-N-acetyltaurinat nach irgendeinem der vorstehenden Ansprüche, wobei der Schritt **a)** und/oder der Schritt **b)** und/oder der Schritt c) und/oder der Schritt **d)** und/oder der Schritt **e)** und/oder der Schritt **f)** und/oder der Schritt **g)** und/oder der Schritt **h)** und/oder der Schritt **i)** bei einer Temperatur von niedriger als oder gleich 50 °C durchgeführt wird.

9. Verfahren zur Herstellung von Magnesium-N-acetyltaurinat nach irgendeinem der vorstehenden Ansprüche, wobei der Schritt **a)** der Solubilisierung unter Vakuum und bei einer Temperatur von niedriger als oder gleich 50 °C durchgeführt wird.

10. Verfahren zur Herstellung von Magnesium-N-acetyltaurinat nach irgendeinem der vorstehenden Ansprüche, wobei die Schritte **a)** der Solubilisierung, **h)** der Reinigung und/oder des Waschens des gekühlten dritten Präzipitats und **i)** der Trocknung/Austrocknung des vierten Präzipitats in Form eines gereinigten und/oder gewaschenen Kuchens unter Vakuum und bei einer Temperatur von niedriger als oder gleich 50 °C durchgeführt werden.

11. Verfahren zur Herstellung von Magnesium-N-acetyltaurinat nach irgendeinem der vorstehenden Ansprüche, wobei die Schritte **a)** der Solubilisierung, **h)** der Reinigung und/oder des Waschens des gekühlten dritten Präzipitats durch Zentrifugieren in Gegenwart einer Entwässerungslösung, vorzugsweise in Gegenwart einer Entwässerungslösung umfassend Ethanol, und **i)** der Trocknung/Austrocknung des vierten Präzipitats in Form eines gereinigten und/oder gewaschenen Kuchens unter Vakuum und bei einer Temperatur von niedriger als oder gleich 50 °C durchgeführt werden.

## Claims

1. Method for manufacturing magnesium N-acetyl-taurinate, in particular magnesium N-acetyl-taurinate dihydrate, comprising:
a) a step of solubilising (i) taurine, (ii) at least one magnesium compound, for example magnesium oxide or magnesium hydroxide and (iii) acetic anhydride in water with salification and acetylation of said taurine in order to obtain a solution comprising taurine magnesium in acetylated form;
b) a step of evaporating and/or distilling said solution obtained in step a) to remove at least some of the excess water and acetic acid and to obtain a first precipitate, in particular a first precipitate in the form of a distillate, comprising the taurine magnesium in acetylated form;
c) at least one step of washing with water, for example by filtration, said first precipitate obtained in step b), said step of washing with water being followed by a step d) of evaporation and/or distillation in order at least partially to remove the excess water and acetic acid and to obtain a second precipitate, in particular a second precipitate in the form of a distillate, comprising the taurine magnesium in acetylated form;
e) at least one step of washing, with a washing solution comprising ethanol, preferably with a washing solution comprising only ethanol, said second precipitate obtained in step d), said step of washing with a washing solution comprising ethanol, preferably with a washing solution comprising only ethanol, being followed by a step f) of evaporation and/or distillation in order at least partially to remove the excess water and ethanol and to obtain a third precipitate comprising the taurine magnesium in acetylated form;
g) a step of cooling the third precipitate obtained in step f);
h) a step of purifying and/or washing said cooled third precipitate obtained in step g) to obtain a fourth precipitate in the form of a cake; and
i) a step of drying/desiccating said fourth precipitate in the form of a purified and/or washed cake obtained in step h).

2. Method for manufacturing magnesium N-acetyl-taurinate according to claim 1, wherein said step a) of solubilising in water is carried out by simultaneous or delayed dissolution of said taurine, said magnesium oxide or said magnesium hydroxide and said acetic anhydride.

3. Method for manufacturing magnesium N-acetyl-taurinate according to claim 1 or 2, wherein said step h) of purifying and/or washing said third precipitate is carried out by filtration in the presence of a filtration solution, preferably in the presence of a filtration solution comprising ethanol.

4. Method for manufacturing magnesium N-acetyl-taurinate according to claim 1 or 2, wherein said step h) of purifying and/or washing said third precipitate is carried out by spin drying in the presence of a dewatering solution, preferably in the presence of a dewatering solution comprising ethanol.

5. Method for manufacturing magnesium N-acetyl-taurinate according to claim 4, wherein said step h) of purifying and/or washing said third precipitate by spin drying is carried out at a spin drying speed of between 500 and 1500 rpm, preferably at a spin drying speed of between 800 and 1200 rpm.

6. Method for manufacturing magnesium N-acetyl-taurinate according to any one of the preceding claims, wherein said solubilisation step a) is carried out in the presence of a solubilisation solution comprising ethanol.

7. Method for manufacturing magnesium N-acetyl-taurinate according to any one of the preceding claims, wherein said step a) and/or said step b) and/or said step d) and/or said step f) and/or said step h) and/or said step i) is carried out under vacuum.

8. Method for manufacturing magnesium N-acetyl-taurinate according to any one of the preceding claims, wherein said step a) and/or said step b) and/or said step c) and/or said step d) and/or said step e) and/or said step f) and/or said step g) and/or said step h) and/or said step i) is carried out at a temperature less than or equal to 50°C.

9. Method for manufacturing magnesium N-acetyl-taurinate according to any one of the preceding claims, wherein said solubilisation step a) is carried out under vacuum and at a temperature less than or equal to 50°C.

10. Method for manufacturing magnesium N-acetyl-taurinate according to any one of the preceding claims, wherein the steps a) of solubilisation, h) of purification and/or washing of said cooled third precipitate and i) of drying/desiccation of said fourth precipitate in the form of a purified and/or washed cake are carried out in vacuum and at a temperature less than or equal to 50°C.

11. Method for manufacturing magnesium N-acetyl-taurinate according to any one of the preceding claims, wherein the steps a) of solubilisation, h) of purification and/or washing of said third precipitate cooled by spin drying in the presence of a dewatering solution, preferably in the presence of a dewatering solution comprising ethanol, and i) of drying/desiccation of said fourth precipitate in the form of a purified and/or washed cake are carried out in vacuum and at a temperature less than or equal to 50°C.
